# EUROPÄISCHE PATENTSCHRIFT

(11) **EP 4 268 648 B1**
(45) Veröffentlichungstag und Bekanntmachung des Hinweises auf die Patenterteilung: **14.08.2024**
(21) Anmeldenummer: 22170087.5
(22) Anmeldetag: 26.04.2022
(51) Int. Cl.: A41D 13/08, A41D 19/00, A41D 19/015, A61B 42/20, A61B 42/10

(54) **SCHUTZEINRICHTUNG FÜR EINE HAND**
PROTECTIVE DEVICE FOR A HAND
DISPOSITIF DE PROTECTION POUR UNE MAIN

(43) Veröffentlichungstag der Anmeldung: 01.11.2023
(73) Patentinhaber: Rogalski, Jakob, 50181 Bedburg (DE)
(72) Erfinder: Rogalski, Jakob, 50181 Bedburg (DE)
(74) Vertreter: Paul & Albrecht Patentanwälte PartG mbB

(56) Entgegenhaltungen:
- US-A- 6 145 128
- US-A1- 2017 215 494
- US-S- D 412 048

## Beschreibung

Die Erfindung betrifft eine Schutzeinrichtung für eine Hand, die genau zwei Fingerschutzelemente und ein die beiden Fingerschutzelemente verbindendes Handflächenschutzelement umfasst, wobei ein Fingerschutzelement für den Daumen und das andere Fingerschutzelement für einen weiteren Finger, insbesondere den Zeigefinger, eines Benutzers bestimmt ist und das Handflächenschutzelement flächig ausgebildet ist, sodass es nur an der Handfläche eines Benutzers in Anlage kommt, der Handrücken des Benutzers hingegen frei oder im wesentlichen frei bleibt.

Ein hoher Anteil gemeldeter Unfälle im Gesundheitsdienst sowie in Laboren ist auf Nadelstich-, Schnitt- und Kratzverletzungen der Haut durch stechende oder schneidende Instrumente zurückzuführen. Die Berufsgenossenschaft für Gesundheitsdienst und Wohlfahrtspflege (BGW) nennt als eine Ursache, dass spitze und scharfe Instrumente wie Kanülen, Lanzetten, Nadeln, Skalpelle und dergleichen nicht unmittelbar nach dem Gebrauch korrekt entsorgt werden. In einigen Fällen verzichten die Benutzer auf das Wiederverschließen mit zugehörigen Sicherungselemente, wie Kappen bzw. Hüllen, und entsorgen die Instrumente offen in einem Abfallbehälter, der dann ein erhebliches Gesundheitsrisiko darstellt.

Es sind "Fingerlinge" bekannt, welche einzeln auf die Fingerspitzen aufgesetzt werden können. Diese Fingerlinge sind jedoch oftmals aus unzureichendem Material, so dass sie im Bereich der Finger keinen ausreichenden Schutz gegen Stich- und Schnittverletzungen bieten. Auch der auf die Finger beschränkte Schutz wird teilweise als unzureichend erachtet.

Aus der DE 43 17 108 A1 geht ein chirurgischer Fingerschutz als Nadelstichschutz hervor. Dieser weist ein Element auf, das flexibel und gegenüber einer Nadelspitze beständig ist. Das Element ist derart ausgebildet, dass es sich an einen Finger anpassen lässt, um eine durchgehende Sperre für einen Nadelstich über wenigstens einen Teil des Fingers bereitzustellen. Das Element hat eine durchgehende, wellenförmige Gestalt, welche mit einer Endkappe endet, die beständig gegenüber einer Nadelspitze ist. Die offenbarten Elemente erstrecken sich, analog zu Fingerlingen, über einzelne Finger.

Aus der US 4,864,661 B geht ein chirurgischer Handschuh aus einem dünnen elastischen Material hervor, der in ausgewählten Bereichen an der Handinnenseite beständig gegen Nagelstiche ausgebildet ist. Hierfür umfasst er in den Bereichen geeignete Materialien, beispielsweise ein Webmaterial oder ein massiv ausgelegtes Material.

Die EP 0 320 541 A1 offenbart einen weiteren chirurgischen Handschuh, der beständig gegen Nadelstiche ist. Er umfasst einen dünnen Basishandschuh aus Latex oder einem synthetischen Gummi, der innenseitig mit einem Verstärkungsmaterial mit einem eng verwobenen stichresistenten Verstärkungsmaterial versehen ist, um das Risiko von Einstichen oder Rissen zu minimieren.

Die DE 20 2006 010 706 U1 offenbart Fingerlinge und einen Teilhandschuh zur Vermeidung von Schnittverletzungen im Friseurhandwerk. Der Teilhandschuh bedeckt zwei Finger des Benutzers, konkret den Zeige- sowie Mittelfinger, die Handinnenfläche, den Handrücken und das Handgelenk. Er besteht aus einem schnittfesten Nitril-Kunststoff oder einem metallischen Gewebe, das kunststoffüberzogen ist.

Die Dokumente US D 412 048 A, US 6 145 128 A und US 2017/215494 A1 offenbaren Schutzeinrichtungen für eine Hand mit jeweils zwei Fingerschutzelementen und einem die beiden Fingerschutzelemente verbindenden Handflächenschutzelement.

Die aus dem Stand der Technik vorbekannten Schutzeinrichtungen haben sich prinzipiell bewährt, um die Hände von Benutzern vor Verletzungen durch spitze und scharfe Gegenstände zu schützen. Es besteht jedoch weiterhin Bedarf an verbesserten Einrichtungen. Insbesondere besteht Bedarf an Schutzeinrichtungen, die eine sehr sichere und gleichzeitig zügige und komfortable Entsorgung von spitzen bzw. scharfen medizinischen Gegenständen nach deren Benutzung ermöglicht.

Es ist daher Aufgabe der vorliegenden Erfindung, eine Schutzeinrichtung der eingangs genannten Art anzugeben, die den Benutzer besonders zuverlässig vor Verletzungen im Bereich der Hand schützt, wenn er mit spitzen bzw. scharfen Gegenständen hantiert, diese insbesondere entsorgt, und die sich gleichzeitig besonders einfach und komfortabel nutzen lässt.

Diese Aufgabe wird bei einer Schutzeinrichtung der eingangs genannten Art dadurch gelöst, dass die Fingerschutzelemente beide näher an einem Endbereich des Handflächenschutzelementes liegen als an einem dem einen Endbereich gegenüberliegenden anderen Endbereich des Handflächenschutzelementes.

Die erfindungsgemäße Schutzeinrichtung bedeckt mit anderen Worten im bestimmungsgemäß an der Hand getragenen Zustand - im Gegensatz zu klassischen "Vollhandschuhe" - nur einen Teil der Hand, konkret zwei Finger zumindest abschnittsweise und die Handinnenfläche. Bei den Fingern, welche die erfindungsgemäße Einrichtung schützt, handelt es sich um den Daumen und einen weiteren Finger, bevorzugt den Zeigefinger. Der Aufbau orientiert sich dabei an den typischen Handbewegungen, die vor allem beim Verschließen bzw. Wiederverschließen von spitzen und scharfen medizinischen Utensilien, wie Kanülen, Lanzetten, Skalpellen und dergleichen, durchgeführt werden. Die Sicherungs- bzw. Verschlusselemente, bei denen es sich z.B. um Hüllen oder Kappen handeln kann, werden in der Regel zwischen dem Daumen und einem weiteren Finger, insbesondere dem Zeigefinger, festgehalten, während das zuvor verwendete Instrument vorsichtig eingeführt wird. Die Anmelderin hat festgestellt, dass hierbei vor allem die Fingerspitzen der beiden für das Festhalten genutzten Finger, aber auch die Handinnenfläche des Benutzers gefährdet sind, verletzt zu werden, etwa in Folge eines Abrutschens oder falschen "Zielens" bei dem Einführvorgang. Durch die erfindungsgemäße Einrichtung werden genau diese besonders gefährdeten Bereiche der Hand bei dem Wiederverschließvorgang zuverlässig geschützt. Gleichzeitig kann die erfindungsgemäße Schutzeinrichtung sehr komfortabel und besonders zügig von einem Benutzer angelegt werden. Er muss nur die Spitzen seines Daumens und eines weiteren Fingers, insbesondere Zeigefingers, in das jeweilige Fingerschutzelement einführen. Das die beiden Fingerschutzelemente verbindende Handflächenschutzelement liegt dann schützend vor seiner Handinnenfläche.

Da die erfindungsgemäße Schutzeinrichtung besonders schnell anlegbar ist, vor allem im Vergleich mit vorbekannten Voll- sowie Teilhandschuhen, die relativ aufwendig über die Hand und bis zum Handgelenk gezogen werden müssen, wird der mit der Entsorgung verbundene Aufwand minimiert. Ist der Aufwand einer sachgemäßen und gleichzeitig sicheren Entsorgung gering, werden Benutzer sich eher für diese entscheiden und die Gefahr von Gesundheitsrisiken wird reduziert. Die erfindungsgemäße Schutzeinrichtung kann dabei auch in Kombination mit dünnen Einweg-Gummihandschuhen genutzt werden, die im medizinischen bzw. pflegerischen Bereich vielfach getragen werden. Diese müssen nicht ausgezogen werden, bevor die erfindungsgemäße Einrichtung angelegt wird. Bei den vorbekannten Voll- bzw. Teilhandschuhen mit Schutzfunktion gegen Einstiche und Schnitte wird dies der Fall sein, da ein Überziehen eines weiteren Voll- oder Teilhandschuhs über einen vorhandenen Gummihandschuh problematisch ist. Da die erfindungsgemäße Schutzeinrichtung kein Ausziehen der Einweghandschuhe voraussetzt, kann der Aufwand weiter reduziert werden.

Dass von den Fingerschutzelementen eines für den Daumen und das andere für einen weiteren Finger, insbesondere den Zeigefinger der Hand eines Benutzers bestimmt ist, bedeutet insbesondere bzw. schließt insbesondere mit ein, dass die Elemente derart ausgebildet und angeordnet sind, dass eines auf den Daumen und das andere auf den weiteren Finger, insbesondere Zeigefinger, der Hand eines Benutzers aufsetzbar ist. Sind die beiden Fingerschutzelemente auf die Finger aufgesetzt, wird die erfindungsgemäße Schutzeinrichtung in bestimmungsgemäßer Weise getragen.

Erfindungsgemäß ist vorgesehen, dass die Fingerschutzelemente beide näher an einem Endbereich des Handflächenschutzelementes liegen als an einem dem einen Endbereich gegenüberliegenden anderen Endbereich des Handflächenschutzelementes. Die beiden Fingerschutzelemente können insbesondere unmittelbar an dem einen Endbereich des Handflächenschutzelements liegen. Alternativ oder zusätzlich kann vorgesehen sein, dass der andere Endbereich, also derjenige Endbereich, an dem die Fingerschutzelemente nicht liegen bzw. von dem sie weiter entfernt sind, ein freier Endbereich des Handflächenschutzelements ist.

Das Handflächenschutzelement ist bevorzugt zungenförmig ausgebildet.

Es kann vorgesehen sein, dass das Handflächenschutzelement einen Endbereich mit U-förmiger Außenkontur, Spitzenbereich genannt, und einen dem Spitzenbereich gegenüberliegenden Endbereich, Wurzelbereich genannt, aufweist. Dann liegen die Fingerschutzelemente beide bevorzugt näher an dem Wurzelbereich als an dem Spitzenbereich. Sie können unmittelbar an dem Wurzelbereich liegen.

Der Spitzenbereich ist bevorzugt ein freier Endbereich des Handflächenschutzelementes.

Besonders bevorzugt ist vorgesehen, dass der Wurzelbereich mit einer dem Spitzenbereich gegenüberliegenden insbesondere geraden Kante abschließt und sich die Fingerschutzelemente bis zu dieser Kante erstrecken. Das Handflächenschutzelement endet dann mit anderen Worten im Bereich der Fingerschutzelemente bzw. erstreckt sich beginnend von den Fingerschutzelementen aus. Diese Ausgestaltung hat sich als ganz besonders geeignet erwiesen, insbesondere für den Fall, dass von den Fingerschutzelementen eines für den Zeigefinger bestimmt ist. Es besteht dann eine ideale Anpassung an den anatomischen Aufbau der menschlichen Hand, bei der Daumen und Zeigefinger beide nahe bzw. an einem Endbereich der Handfläche angeordnet sind. Bei der linken Hand liegen diese, wenn man auf die Handinnenfläche blickt, konkret am linken Endbereich der Handfläche und bei der rechten Hand am rechten.

Weiterhin kann vorgesehen sein, dass die beiden Fingerschutzelemente in Bezug auf die beiden gegenüberliegenden Endbereiche des Handflächenschutzelementes seitlich des Handflächenschutzelementes angeordnet sind, bevorzugt seitlich von dem Handflächenschutzelement abragen. In Bezug auf die beiden gegenüberliegenden Endbereiche seitlich bedeutet insbesondere in Bezug auf eine diese beiden Endbereiche verbindende gedachte Linie seitlich des Handflächenschutzelementes.

Als ganz besonders vorteilhaft hat es sich ferner erwiesen, wenn von den beiden Fingerschutzelementen je eines an zwei gegenüberliegenden Seiten des Handflächenschutzelementes angeordnet ist.

Alternativ oder zusätzlich kann vorgesehen sein, dass die Fingerschutzelemente an einem Ende offen und an ihrem dem offenen Ende gegenüber liegenden anderen Ende geschlossen sind, wobei das offene Ende dem Handflächenschutzelement zugewandt und das geschlossene Ende von dem Handflächenschutzelement abgewandt ist. Dann kann man auf besonders komfortable Weise die Spitzen von Daumens und einem weiteren Finger, insbesondere Daumen und Zeigefinger, einer Hand in die Fingerschutzelemente einführen, um die erfindungsgemäße Schutzeinrichtung anzulegen. Hierfür kann man Daumen und Zeigefinger z.B. V-förmig auseinanderstellen.

Das Handflächenschutzelement liegt dann zwischen den beiden Fingerschutzelementen und sein einer Endbereich liegt im getragenen Zustand insbesondere auf demjenigen Endbereich der Handfläche eines Benutzers, welcher Daumen und Zeigefinger miteinander verbindet und erstreckt sich von dort beginnend bevorzugt über die gesamte Handfläche des Benutzers.

In weiterer besonders bevorzugter Ausführungsform der erfindungsgemäßen Schutzeinrichtung ist vorgesehen, dass die Fingerschutzelemente jeweils eine Fingerschutzkappe mit einer geschlossenen Oberseite und einer offenen Unterseite umfassen. Es ist auch möglich, dass die Fingerschutzelemente durch derartige Fingerschutzkappen gebildet werden. Fingerschutzkappen können auf einfache Weise auf den Daumen und den weiteren Finger eines Benutzers aufgesetzt werden und insbesondere die Spitzen dieser Finger zuverlässig schützen.

Die Fingerschutzkappen zeichnen sich bevorzugt zumindest im Wesentlichen durch die Form eines Hohlkegelstumpfes oder zumindest im Wesentlichen durch die Form eines einseitig geschlossenen Hohlzylinders aus. Diese Formen haben sich für auf Finger aufsetzbare Kappen besonders bewährt. Unter zumindest im Wesentlich ist dabei zu verstehen, dass Abweichungen der genauen Hohlkegelstumpf- bzw. Hohlzylinderform vorliegen können. So können die Fingerschutzkappen insbesondere abgerundet sein, z.B. abgerundete Kanten und gekrümmte, insbesondere konvexe Stirnwandung aufweisen, wie es von Fingerhüten bekannt ist.

Auch kann vorgesehen sein, dass sich die Fingerschutzkappen in ihren Stirnseiten durch eine größere Materialstärke auszeichnen als im verbleibenden Bereich. Durch die stirnseitige Verstärkung des Materials wird in Risikozonen besonders viel Widerstandsfähigkeit geboten. Unter den Stirnseiten liegen im getragenen Zustand die Fingerkuppen, die besonders gefährdet sind, vor allem, was Einstiche angeht.

Eine weitere Ausführungsform der Erfindung zeichnet sich dadurch aus, dass die Fingerschutzelemente einen Fingerschutzsteg aufweisen. Ist ein solcher vorhanden, schließt er sich insbesondere an die offene Unterseite der Fingerschutzkappe des jeweiligen Fingerschutzelementes an. Ein Fingerschutzsteg kann insbesondere jeweils dem Schutz eines unteren, der Handinnenfläche näheren Abschnitts von Daumen und weiterem Finger, insbesondere Zeigefinger dienen.

Bei dieser Ausführungsform werden die oberen Abschnitte des Daumens und des weiteren Fingers insbesondere rundum verdeckt und geschützt, während die unteren Abschnitte der Finger nur an ihrer Innenseite durch den jeweiligen Fingerschutzsteg geschützt sind, jedoch außen- bzw. oberseitig und seitlich frei bleiben. Die Fingerschutzstege sind entsprechend bevorzugt ausgestaltet und angeordnet, um einen Längsabschnitt des jeweiligen Fingers insbesondere nur innenseitig zu verdecken und somit zu schützen. Die Fingerschutzstege sind bevorzugt flächig ausgebildet und kommen nur an den Innenseiten der Finger in Anlage, während sie die Finger außen- bzw. oberseitig und seitlich frei lassen.

Umfassen die Fingerschutzelemente jeweils einen Fingerschutzsteg, gilt bevorzugt, dass der Fingerschutzsteg des für den Daumen bestimmten Fingerschutzelementes eine geringere Länge aufweist als der Fingerschutzsteg des für den weiteren Finger, insbesondere Zeigefinger bestimmten Fingerschutzelementes.

Eine weitere besonders bevorzugte Ausgestaltung zeichnet sich ferner dadurch aus, dass die Fingerschutzelemente ausschließlich über das Handflächenschutzelement miteinander verbunden und insbesondere einteilig ausgebildet sind. Umfassen die Fingerschutzelemente Fingerschutzkappen und sich daran anschließende Fingerschutzstege, ist das Handflächenschutzelement bevorzugt mit den Fingerschutzstegen verbunden.

Zusätzlich kann ein Befestigungsband vorgesehen sein, dessen eines Ende mit dem Handflächenschutzelement fest verbunden ist, und zwar bevorzugt in dem Bereich, der im bestimmungsgemäßen Zustand zwischen der Wurzel des Daumens und der Wurzel des weiteren Fingers gelegen ist, und dessen freies Ende an dem Handflächenschutzelement, insbesondere an dessen Bereich welcher an dem Handballen eines Benutzers zugeordnet ist, beispielsweise mittels eines Klettverschlusses lösbar fixiert werden kann, nachdem das Befestigungsband über den Handrücken des Benutzers gelegt wurde. Auf diese Weise kann das Handflächenschutzelement zusätzlich an der Hand fixiert werden.

An der Innenseite und/oder der Außenseite der Fingerschutzkappen können Noppen vorgesehen sein, wodurch ein besonders sicheres Handling spitzer bzw. scharfer Utensilien möglich wird. Innenseitig der Fingerschutzkappen vorhandene Noppen gewährleisten vor allem einen besonders sicheren Halt der erfindungsgemäßen Einrichtung bzw. der Fingerschutzkappen an der Hand eines Benutzers. Außenseitige Noppen ermöglichen vor allem mehr Kontrolle, was zu hantierende Gegenstände angeht.

Die Materialstärke der erfindungsgemäßen Schutzeinrichtung ist zweckmäßigerweise derart gewählt, dass ein ausreichendes Maß an Stich- bzw. Schnittfestigkeit gewährleistet wird.

Als besonders geeignet hat es sich erwiesen, wenn die Materialstärke der Fingerschutzkappen zumindest abschnittsweise mindestens 1,0 mm, insbesondere mindestens 2,0 mm beträgt und/oder die Materialstärke der Fingerschutzkappen maximal 5,0 mm, insbesondere maximal 4,0 mm beträgt und bevorzugt 3,0 mm beträgt.

Alternativ und/oder zusätzlich kann vorgesehen sein, dass die Materialstärke der Fingerschutzstege zumindest im Bereich der den Fingerkuppen zugeordneten vorderen Enden mindestens 1,0 mm, insbesondere mindestens 2,0 mm beträgt und/oder maximal 5,0 mm, insbesondere maximal 4,0 mm beträgt und bevorzugt 3,0 ±0,5 mm beträgt.

In weiterer Ausgestaltung der Erfindung kann der Innenraum der Fingerschutzkappen insbesondere an deren breitesten Stelle einen Durchmesser von 1,5 bis 3,0 cm und/oder eine Länge im Bereich von 1,5 bis 6,0 cm aufweisen. Besonders bevorzugt ist, wenn die Fingerschutzkappen so ausgestaltet sind, dass Sie nur das jeweils vordere Daumen- bzw. Fingerglied bis zum ersten Fingergelenk abdecken. In diesem Fall besitzen die Fingerschutzkappen eine Länge von wenigstens 2,0 cm, insbesondere wenigstens 2,5 cm und/oder von höchstens 4,0 cm, insbesondere höchstens 3,0 cm.

Das Handflächenschutzelement ist zweckmäßigerweise derart ausgestaltet und angeordnet, dass es sich im bestimmungsgemäß getragenen Zustand der Schutzeinrichtung, wenn die beiden Fingerschutzelemente jeweils auf einen Finger, insbesondere den Daumen und Zeigefinger eines Benutzers aufgesetzt sind, schützend vor der Handinnenfläche des Benutzers erstreckt. Es kann als flexible Matte ausgestaltet sein, die zwischen den beiden Fingerschutzelementen liegt und im getragenen Zustand die Handinnenfläche bedeckt und an dieser anliegt.

Die erfindungsgemäße Schutzeinrichtung bzw. deren Fingerschutzelemente und/oder Handflächenschutzelement umfassen bevorzugt ein elastisch verformbares Material, insbesondere wenigstens ein Elastomer oder Silikon bzw. bestehen daraus. Alternativ oder zusätzlich können die Fingerschutzelemente und/oder das Handflächenschutzelement Latex umfassen bzw. daraus bestehen. Wichtig ist, dass das Handflächenschutzelement ausreichend flexibel ist, sodass es sich im Gebrauch an der Handinnenfläche anlegt. Ebenso müssen die Fingerschutzstege so flexibel sein, dass der Daumen und der weitere Finger frei bewegt werden können. Dies wird eine durch entsprechende Abstimmung der Elastizität des verwendeten Materials und der Materialdicke sichergestellt. Das Material kann verstärkt sein. Insbesondere können Verstärkungselemente wie beispielsweise Streifen aus Metallblech, Kunststoff oder der gleichen oder auch Verstärkungsfasern in das Material eingebettet sein.

Eine weitere Ausgestaltung der Erfindung zeichnet sich dadurch aus, dass das Handflächenschutzelement eine Dicke von wenigstens 1,0 mm, insbesondere wenigstens 2,0 mm und/oder maximal 5,0 mm, insbesondere maximal 4,0 mm aufweist, wobei die Dicke bevorzugt 3,0 ± 0,5 mm aufweist, und/oder dass das Handflächenschutzelement eine Länge von mindestens 6,0 cm, insbesondere mindestens 7,0 cm und/oder von höchstens 11,0 cm, insbesondere höchstens 9,0 ± 0,5 cm aufweist.

In weiterer zweckmäßiger Ausgestaltung ist die erfindungsgemäße Schutzeinrichtung derart ausgebildet, dass sie drei Finger, bevorzugt den Ringfinger, Mittelfinger und kleinen Finger, und/oder den Handrücken und/oder das Handgelenk frei lässt, wenn ein Benutzer diese in bestimmungsgemäßer Weise trägt. Als ganz besonders geeignet hat sich erwiesen, wenn sie drei Finger, insbesondere der Ringfinger, Mittelfinger und kleiner Finger, und den Handrücken und das Handgelenk freilässt. Besonders bevorzugt werden nur die oberen Endbereiche zweier Finger durch die beiden Fingerschutzkappen und die Handinnenfläche durch das Handflächenschutzelement bedeckt.

Hinsichtlich der Ausgestaltung der Erfindung wird auch auf die Unteransprüche sowie auf die nachfolgende Beschreibung eines Ausführungsbeispiels unter Bezugnahme auf die beiliegende Zeichnung verwiesen.

In der Zeichnung zeigt in rein schematischer Darstellung:
- Figur 1: ein Ausführungsbeispiel einer erfindungsgemäßen Schutzeinrichtung im bestimmungsgemäß an der Hand eines Benutzers getragenen Zustand in einer Ansicht auf die Handinnenfläche;
- Figur 2: die Schutzeinrichtung aus Figur 1 im getragenen Zustand in einer Ansicht auf den Handrücken:
- Figur 3: die Schutzeinrichtung aus Figur 1 im getragenen Zustand in einer Ansicht auf die Handinnenfläche, wobei Daumen und Zeigefinger und die darauf aufgesetzten Fingerschutzkappen zusammengeführt sind, um einen Gegenstand zu halten;
- Figur 4: die Schutzeinrichtung aus Figur 1 im getragenen Zustand in einer Ansicht auf die Handinnenfläche, wobei Daumen und Zeigefinger und die darauf aufgesetzten Fingerschutzkappen zusammengeführt sind, um einen Gegenstand zu halten;
- Figur 5: die Schutzeinrichtung aus Figur 1 im getragenen Zustand von der Seite;
- Figur 6: die Schutzeinrichtung aus Figur 1 im getragenen Zustand in einer Ansicht auf die Handinnenfläche, wobei die Spitzen von Mittelfinger, Ringfinger und kleinem Finger des Benutzers hinter dem Handflächenschutzelement positioniert sind;
- Figur 7: die Ansicht aus Figur 6, wobei Daumen und Zeigefinger näher zusammengeführt sind;
- Figur 8: die Schutzeinrichtung aus Figur 1 im ungetragenen, aufgeklappten Zustand in der Aufsicht; und
- Figur 9: eine Schnittdarstellung der Schutzeinrichtung entlang der Linie AA in Figur 8.

In den Figuren sind gleiche Komponenten bzw. Elemente mit gleichen Bezugszeichen versehen.

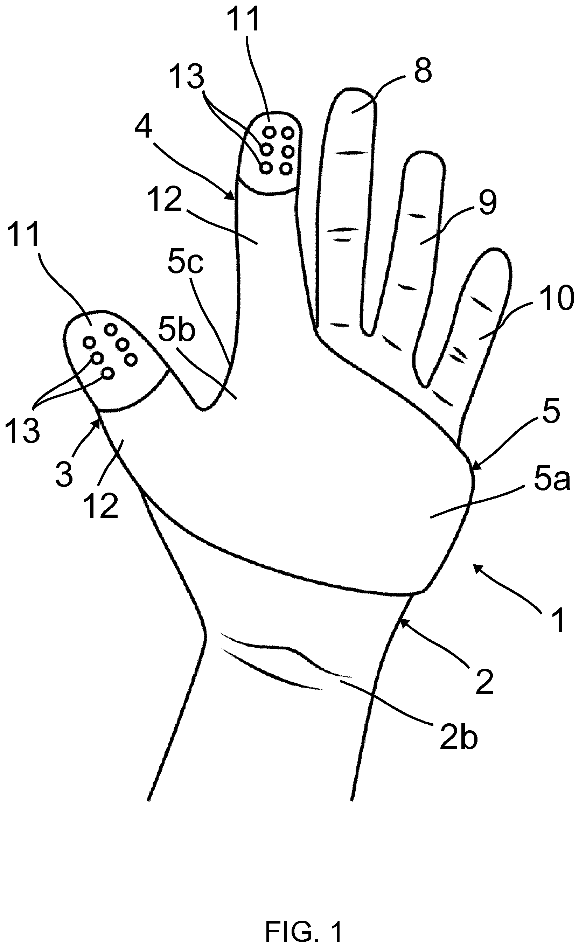

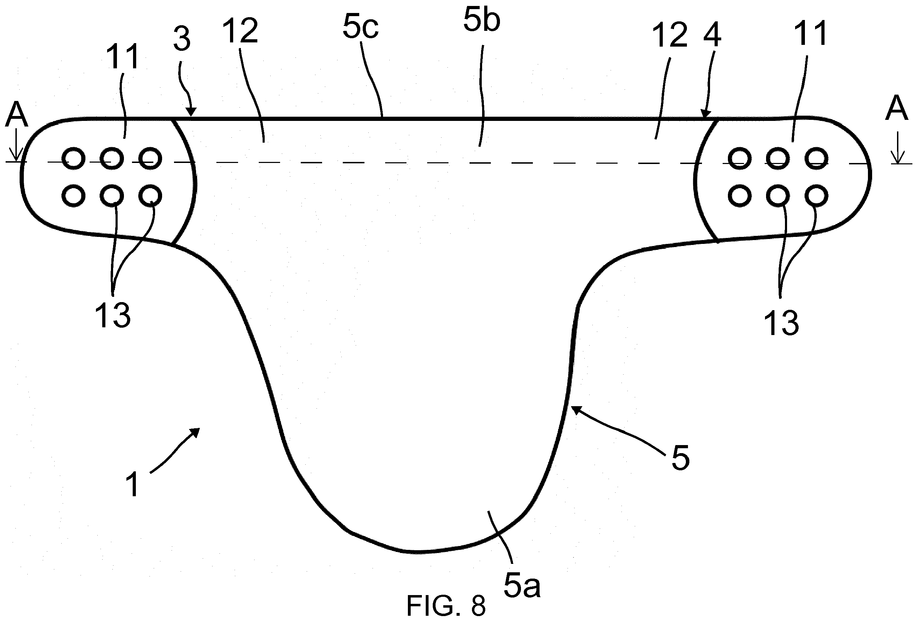

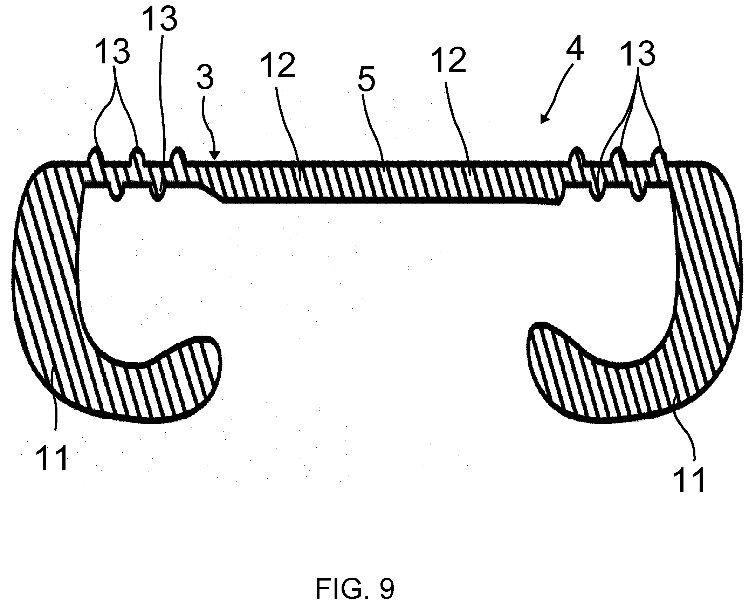

Die Figuren 1 bis 9 zeigen verschiedene Ansichten eines Ausführungsbeispiels einer erfindungsgemäßen Schutzeinrichtung 1. Dabei zeigen die Figuren 1 bis 7 die Einrichtung 1 im an der Hand 2 eines Benutzers getragenen Zustand, während sie den Figuren 8 und 9 im ungetragenen Zustand in Alleinstellung entnommen werden kann.

Die erfindungsgemäße Schutzeinrichtung 1 umfasst zwei Fingerschutzelemente 3, 4 und ein Handflächenschutzelement 5, welches die beiden Fingerschutzelemente 3, 4 miteinander verbindet. Dabei ist ein Fingerschutzelement 3 erkennbar für den Daumen 6 und das andere Fingerschutzelement 4 erkennbar für den Zeigefinger 7 der Hand 2 eines Benutzers bestimmt.

Die Schutzeinrichtung 1 umfasst genau zwei Fingerschutzelemente 3, 4. Für die verbleibenden Finger, also den Mittelfinger 8, Ringfinger 9 und kleinen Finger 10 weist die Schutzeinrichtung 1 keine Fingerschutzelemente auf. Diese Finger 8, 9, 10 bleiben entsprechend frei, wenn die Einrichtung 1 an der Hand 2 eines Benutzers getragen wird. Gleiches gilt für den Handrücken 2a und das Handgelenk 2b, die ebenfalls frei bleiben. Die Schutzeinrichtung 1 weist auch für diese keine Schutzelemente auf.

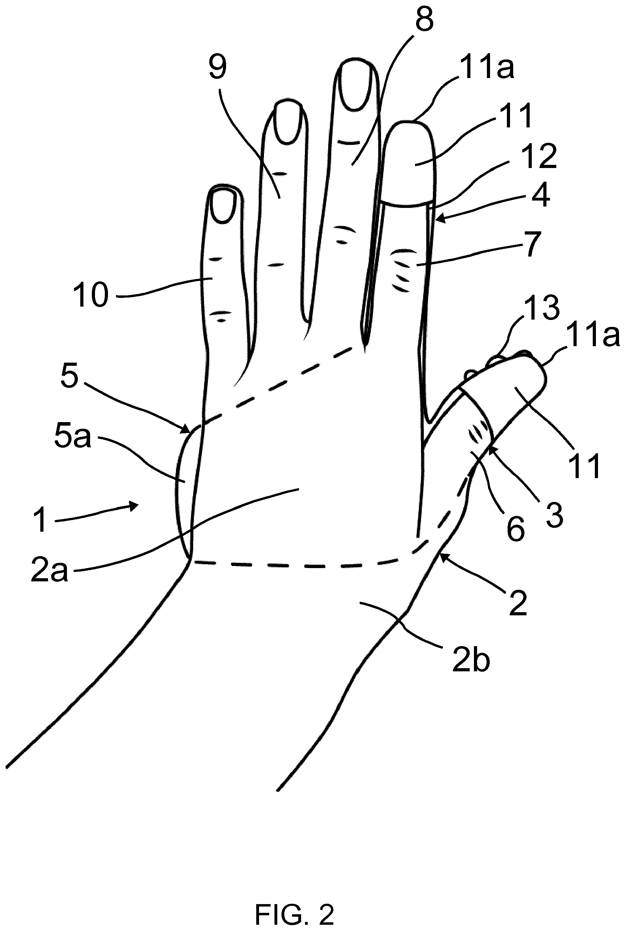

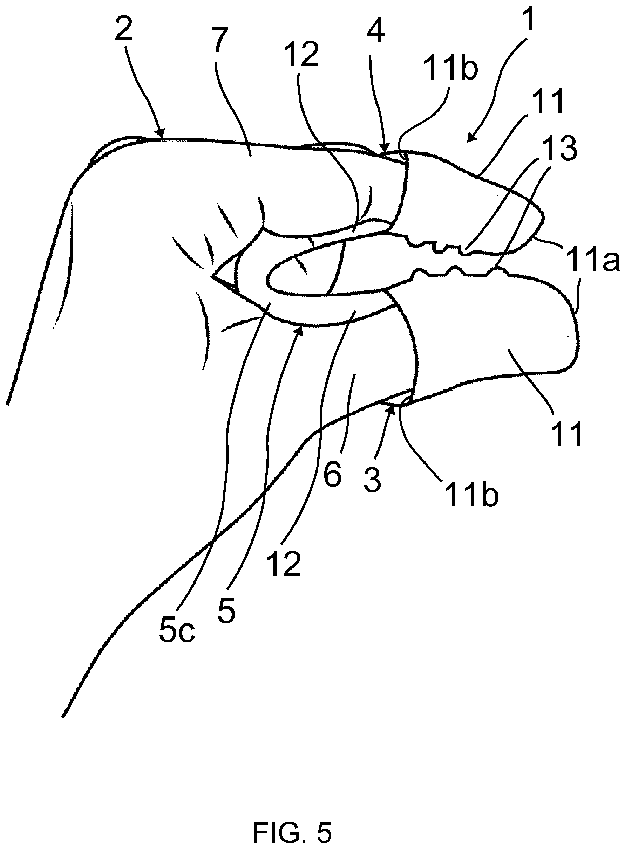

Die beiden Fingerschutzelemente 3, 4 für den Daumen 6 und den Zeigefinger 7 umfassen bei dem dargestellten Ausführungsbeispiel jeweils eine Fingerschutzkappe 11 und einen Fingerschutzsteg 12. Die Fingerschutzkappen 11 entsprechen in ihrer Form konventionellen Fingerhüten, die auch beim Nähen zum Einsatz kommen. Sie haben in etwa die Form von Hohlkegelstümpfen, wobei der stirnseitige Endabschnitt dieser erkennbar abgerundet und somit an die Form der aufzunehmenden Fingerspitzen angepasst ist. Die Kappen 11 sind an ihrer Stirnseite 11a verschlossen und ihrer der Stirnseite gegenüberliegenden Unterseite 11b offen ausgebildet, so dass sie auf die Fingerspitze aufgesetzt werden können (vgl. insbesondere die Figuren 2 und 5, welche den aufgesetzten Zustand besonders gut zeigen).

Wie man erkennt, ist die Länge der Fingerschutzkappen 11 derart gewählt, dass von diesen im getragenen Zustand nur ein oberer Abschnitt des Daumens 6 bzw. Zeigefingers 7 aufgenommen und bedeckt wird, konkret nur derjenige Abschnitt, der sich von der Fingerspitze bis etwa zum obersten Gelenk des jeweiligen Fingers 6, 7, erstreckt. Den restlichen Abschnitt von Daumen und Zeigefinger bis zur Handfläche wird von den Fingerschutzkappen 11 freigelassen. Die Länge des Innenraums der Fingerschutzkappen 11 liegt hier bei 3,0 cm. Der Durchmesser von deren Innenraum beträgt an der breitesten Stelle 2,5 cm. Diese Werte sind rein beispielhaft zu verstehen. Zweckmäßiger Weise kommt die erfindungsgemäße Schutzeinrichtung 1 - genau wie konventionelle Handschuhe - in verschiedenen Größen für verschieden große Hände zum Einsatz.

An die Fingerschutzkappen 11 schließt sich nach unten hin, mit anderen Worten beginnend an deren offenen Unterseiten, jeweils ein Fingerschutzsteg 12 an, welcher den Daumen 6 und Zeigefinger 7 über den verbleibenden, nicht von der Fingerschutzkappe 11 verdeckten Längsabschnitt des Fingers 6, 7, der sich bis zur Mittelhand erstreckt, schützt, jedoch nur im Bereich der Innenfläche des jeweiligen Fingers 6, 7. Sie sind entsprechend ausgestaltet und angeordnet, um den unteren Längsabschnitt des jeweiligen Fingers 6, 7, der sich vom obersten Fingergelenk bis zum unteren Enden des Fingers 6, 7 erstreckt, nur innenseitig zu verdecken und zu schützen. Außen- bzw. rückseitig und seitlich lassen sie den Daumen 6 und Zeigefinger 7 frei. Die Fingerschutzstege 12 haben hier einen etwa rechteckigen Außenumfang. Sie weisen beide eine Breite von 2,0 cm auf, haben jedoch unterschiedliche Längen. Der Fingerschutzsteg 12 des für den Daumen 6 bestimmten Fingerschutzelementes 3 ist kürzer als der Fingerschutzsteg des für den Zeigefinger 7 bestimmten Fingerschutzsteges 12, um den verschiedenen Längen dieser beiden Finger Rechnung zu tragen.

Die Fingerschutzstege 12 sind einteilig mit den Fingerschutzkappen 11 ausgebildet und an diese angeformt. Die Fingerschutzstege 12 und Kappen 11 bestehen aus einem elastischen Kunststoffmaterial, insbesondere einem Elastomer oder Silikon. Sie weisen eine geeignete Materialstärke auf, um einen guten Schutz gegen Einstiche, Kratzer, Schnitte und dergleichen zu bieten. Bei dem dargestellten Beispiel beträgt die Materialstärke im Bereich der Stirnseiten 11a der Fingerschutzkappen 11 5,0 mm, während sie in den verbleibenden Bereichen der Fingerschutzkappen 11 3,0 mm beträgt. Die Stirnseiten 11a der Fingerschutzkappen 11 sind also verstärkt, um in den dortigen Risikozoneneinen besonders guten Schutz zu bieten. Die größere Materialstärke ist in der Schnittdarstellung in Figur 9 zu erkennen.

Die Materialstärke der Fingerschutzstege 12 liegt bei 3,0 mm.

Die Fingerschutzelemente 3, 4 weisen Noppen 13 auf und zwar in den zu den Fingerschutzstegen 12 benachbarten beziehungsweise an diese angrenzenden Bereiche der Fingerschutzkappen 11. Die Noppen 13 sind dabei sowohl innen- als auch außenseitig an diesen Bereichen der Fingerschutzkappen 11 vorgesehen. Außenseitig sind die Noppen 13 an derjenigen Seite der Fingerschutzkappen 11 positioniert, an die sich auch jeweils der Fingerschutzsteg 12 anschließt, und die dem Handflächenschutzelement 5 zugewandt ist. Innenseitig sind Noppen 13, die den äußeren Noppen 13 gegenüberliegend, aber seitlich versetzt zu diesen, angeordnet. In den Bereichen, in denen Noppen 13 vorgesehen sind, ist die Materialstärke der Fingerschutzkappen 4 etwas reduziert, so dass die Spitzen der inneren Noppen 13 etwa bindig mit der Innenseite der Fingerschutzstege 12 abschließen. Die inneren Noppen 13 gewährleisten vor allem einen besonders sicheren Halt der erfindungsgemäßen Einrichtung bzw. der Fingerschutzkappen 11 an der Hand 2 eines Benutzers und die äußeren Noppen 13 vor allem mehr Kontrolle, was zu hantierende Gegenstände angeht.

Das Handflächenschutzelement 5, das hier zungenförmig ausgebildet ist, verbindet die beiden Fingerschutzelemente 3, 4 der Einrichtung 1, konkret deren Fingerschutzstege 12 miteinander. Die Fingerschutzelemente 3, 4, d.h. die Fingerschutzstege 12 dieser sind dabei ausschließlich über das Handflächenschutzelement 5 miteinander verbunden.

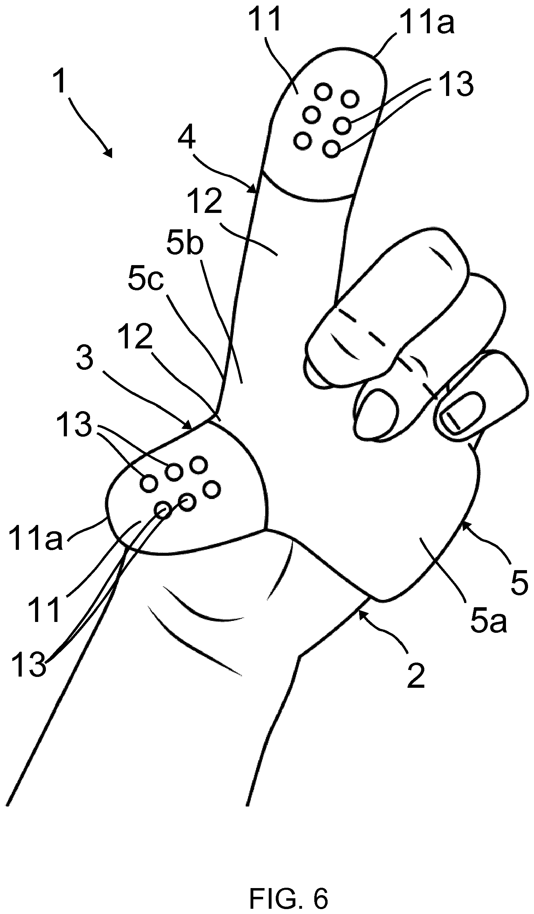

Das Handflächenschutzelement 5 ist flächig als eine flexible Matte ausgebildet. Es weist einen freien Endbereich mit U-förmiger Außenkontur, einen Spitzenbereich 5a, und einen dem Spitzenbereich 5a gegenüberliegenden Endbereich, Wurzelbereich 5b, aufweist (vgl. insbesondere die Figuren 6 und 8). Die beiden Fingerschutzelemente 3, 4 liegen dabei beide näher an dem Wurzelbereich 5b als an dem Spitzenbereich 5a. Bei dem gezeigten Beispiel liegen sie unmittelbar an dem Wurzelbereich 5b. Der Wurzelbereich 5b schließt mit einer dem Spitzenbereich 5a gegenüberliegenden geraden Kante 5c ab, und die Fingerschutzelemente 3, 4, konkret deren Fingerschutzstege 12, erstrecken sich bis zu dieser Kante 5c.

Wie man in den Figuren erkennt, liegen die beiden Fingerschutzelemente 3, 4 ferner in Bezug auf die beiden gegenüberliegenden Endbereiche 5a und 5b seitlich des Handflächenschutzelementes 5 und zwar jeweils eines an zwei gegenüberliegenden Seiten dieses. Sie ragen hier seitlich von dem Handflächenschutzelement 5 ab. Die beiden Fingerschutzelemente 3, 4 fluchten.

Das offene Ende der Fingerschutzelemente 3, 4 und das offene Ende der jeweiligen Fingerschutzkappen 11 sind dem Handflächenschutzelement 5 zugewandt, und die geschlossenen Stirnseiten der Fingerschutzelemente 3, 4, konkret von deren Fingerschutzkappen 11, sind von dem Handflächenschutzelement 5 abgewandt. So können auf besonders komfortable Weise die Spitzen von Daumen 6 und Zeigefinger 7 in die Fingerschutzelemente 3, 4 und deren Kappen 11 eingeführt werden, um die Schutzeinrichtung 1 anzulegen.

Wie man in den Figuren erkennt, ist das Handflächenschutzelement 5 derart ausgestaltet und angeordnet, dass es sich im bestimmungsgemäß getragenen Zustand der Schutzeinrichtung 1, wenn die beiden Fingerschutzelemente 3, 4 bzw. deren Kappen 11 auf Daumen 6 und Zeigefinger 7 eines Benutzers aufgesetzt sind, schützend vor der Handinnenfläche des Benutzers erstreckt. Es weist hier eine Länge von 8,0 cm auf (Ausdehnung von der Kante 5c bis zum Ende des Spitzenbereichs 5a).

Das Handflächenschutzelement 5 und die Fingerschutzelemente 3, 4 sind einteilig ausgebildet. Das Handflächenschutzelement 5 besteht aus dem gleichen Material wie die Fingerschutzelemente 3, 4 und ist 3,0 mm dick.

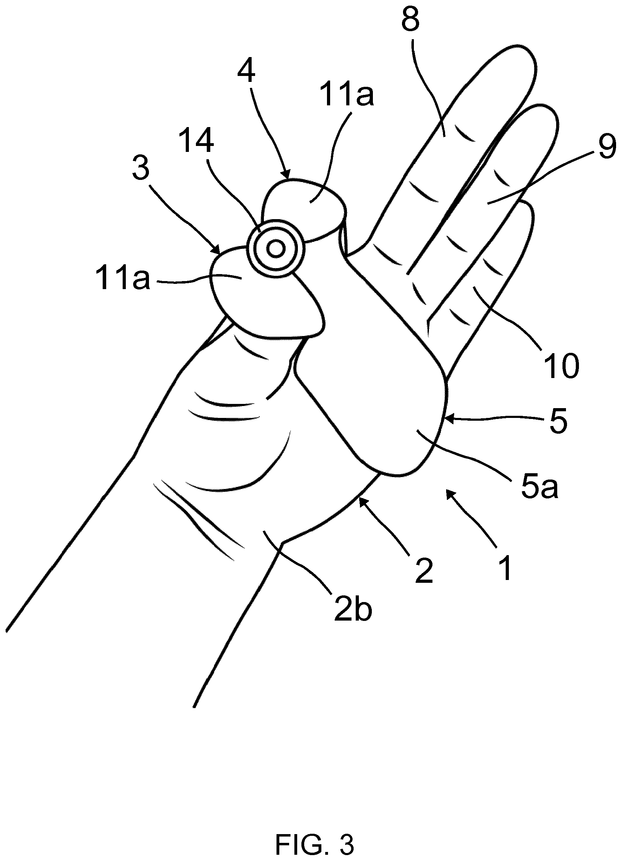

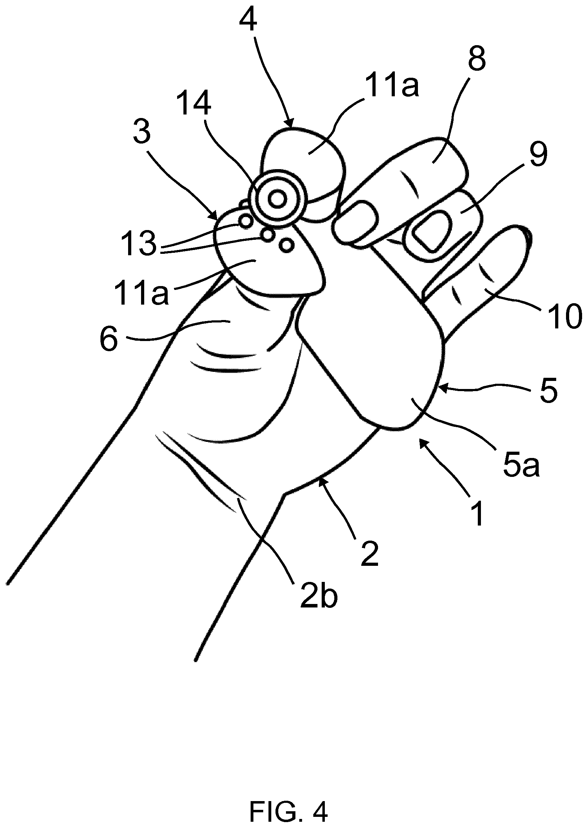

Der Aufbau der Schutzeinrichtung 1 orientiert sich an den typischen Handbewegungen, die beim Verschließen bzw. Wiederverschließen von spitzen und scharfen medizinischen Utensilien, wie Kanülen, Lanzetten, Skalpellen und dergleichen, durchgeführt werden. Die Sicherungs- bzw. Verschlusselemente, bei denen es sich z.B. um Hüllen oder Kappen 14 handeln kann, werden in der Regel zwischen dem Daumen 6 und Zeigefinger 7 festgehalten (vgl. die Figuren 3 und 4), während das zuvor verwendete Instrument vorsichtig eingeführt wird. Die Anmelderin hat festgestellt, dass hierbei insbesondere die Fingerspitzen der beiden für das Festhalten genutzten Finger 6, 7 und die Handinnenfläche des Benutzers gefährdet sind, verletzt zu werden. Durch die Schutzeinrichtung 1 werden genau diese besonders gefährdeten Bereiche der Hand 2 bei dem Wiederverschließvorgang zuverlässig geschützt. Gleichzeitig kann die Schutzeinrichtung 1 sehr komfortabel und besonders zügig von einem Benutzer angelegt werden. Er muss nur die Spitzen seines Daumens 6 und Zeigefingers 7 die Fingerschutzkappen 11 der Fingerschutzelemente 3, 4 einschieben. Das die beiden Fingerschutzelemente 3, 4 verbindende Handflächenschutzelement 5 liegt dann schützend vor seiner Handinnenfläche. Da die Schutzeinrichtung 1 aus einem elastischen Material besteht, können die Fingerschutzkappen 3, 4 komfortabel zusammengeführt werden, um z.B. eine Kappe 14 zu greifen. Die Fingerschutzstege 12 und das Handflächenschutzelement 5 werden dann entsprechend gebogen, was insbesondere in der Figur 5 erkennbar ist.

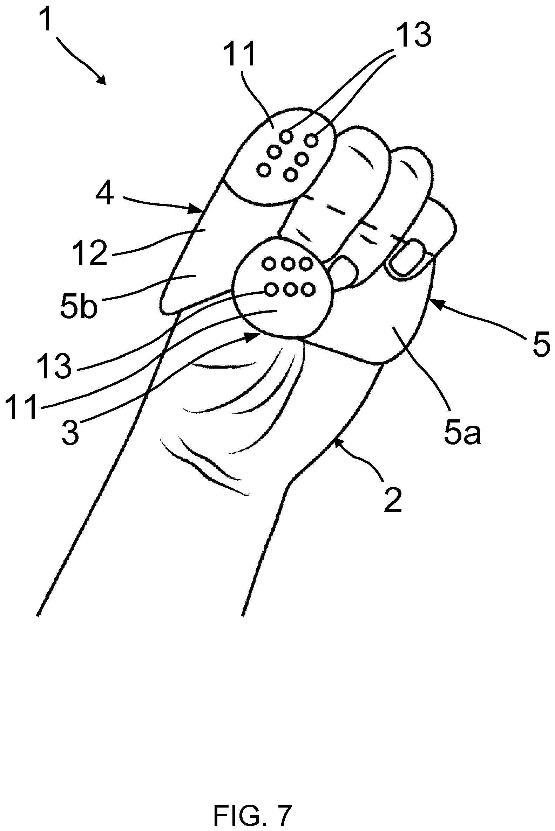

Der Benutzer kann wahlweise diejenigen Finger, die nicht von einem Fingerschutzelement 3, 4 bedeckt sind, also den Mittelfinger 8, Ringfinger 9 und kleinen Finger 10, bzw. deren vordere Abschnitte, hinter das Handflächenschutzelement 5 schieben, um auch diese zu schützen. Dies ist in den Figuren 6 und 7 schematisch angedeutet.

Da die Schutzeinrichtung 1 besonders schnell anlegbar ist, vor allem im Vergleich mit vorbekannten Voll- sowie Teilhandschuhen, die relativ aufwendig über die Hand und bis zum Handgelenk gezogen werden müssen, wird der mit der Entsorgung verbundene Aufwand minimiert. Ist der Aufwand einer sachgemäßen und gleichzeitig sicheren Entsorgung gering, werden Benutzer sich eher für diese entscheiden und die Gefahr von Gesundheitsrisiken wird reduziert. Die Schutzeinrichtung 1 kann dabei auch in Kombination mit dünnen Einweg-Gummihandschuhen genutzt werden, die im medizinischen bzw. pflegerischen Bereich vielfach getragen werden. Diese müssen nicht ausgezogen werden, bevor die Schutzeinrichtung 1 durch Einschieben von Daumen 6 und Zeigefinger 7, bzw. Spitzen dieser, angelegt wird.

### Bezugszeichenliste

- 1: Schutzeinrichtung
- 2: Hand
- 2a: Handrücken
- 2b: Handgelenk
- 3: Fingerschutzelement
- 4: Fingerschutzelement
- 5: Handflächenschutzelement
- 5a: Spitzenbereich
- 5b: Wurzelbereich
- 6: Daumen
- 7: Zeigefinger
- 8: Mittelfinger
- 9: Ringfinger
- 10: kleiner Finger
- 11: Fingerschutzkappe
- 11a: Stirnseite
- 11b: Unterseite
- 12: Fingerschutzsteg
- 13: Noppe
- 14: Kappe

## Patentansprüche

1. Schutzeinrichtung (1) für eine Hand (2), die genau zwei Fingerschutzelemente (3, 4) und ein die beiden Fingerschutzelemente (3, 4) verbindendes Handflächenschutzelement (5) umfasst, wobei ein Fingerschutzelement (3) für den Daumen (6) und das andere Fingerschutzelement (4) für einen weiteren Finger, insbesondere den Zeigefinger (7), eines Benutzers bestimmt ist und **dadurch gekennzeichnet, dass** das Handflächenschutzelement (5) flächig ausgebildet ist, sodass es nur an der Handfläche eines Benutzers in Anlage kommt, der Handrücken des Benutzers hingegen frei oder im wesentlichen frei bleibt, wobei die Fingerschutzelemente (3, 4) beide näher an einem Endbereich (5b) des Handflächenschutzelementes (5) liegen als an einem dem einen Endbereich (5b) gegenüberliegenden anderen Endbereich (5a) des Handflächenschutzelementes (5).

2. Schutzeinrichtung (1) nach Anspruch 1, **dadurch gekennzeichnet, dass** die Fingerschutzelemente (3, 4) beide unmittelbar an dem einen Endbereich (5b) des Handflächenschutzelementes (5) liegen, und/oder dass der andere Endbereich (5a) ein freier Endbereich des Handflächenschutzelementes (5) ist.

3. Schutzeinrichtung (1) nach Anspruch 2, **dadurch gekennzeichnet, dass** das Handflächenschutzelement (5) einen bevorzugt freien Endbereich mit U-förmiger Außenkontur, Spitzenbereich (5a), und einen dem Spitzenbereich (5a) gegenüberliegenden Endbereich, Wurzelbereich(5b), aufweist, wobei die Fingerschutzelemente (3, 4) beide näher an dem Wurzelbereich (5b) liegen als an dem Spitzenbereich (5a), insbesondere, wobei die Fingerschutzelemente (3, 4) beide unmittelbar an dem Wurzelbereich (5b) liegen, bevorzugt, wobei der Wurzelbereich (5b) mit einer dem Spitzenbereich (5a) gegenüberliegenden insbesondere geraden Kante (5c) abschließt und sich die Fingerschutzelemente (3, 4) bis zu dieser Kante (5c) erstrecken.

4. Schutzeinrichtung (1) nach Anspruch 2 oder 3, **dadurch gekennzeichnet, dass** die Fingerschutzelemente (3, 4) in Bezug auf die beiden gegenüberliegenden Endbereiche (5a, 5b) seitlich des Handflächenschutzelementes (5) angeordnet sind, bevorzugt seitlich von dem Handflächenschutzelement (5) abragen.

5. Schutzeinrichtung (1) nach Anspruch 4 **dadurch gekennzeichnet, dass** von den Fingerschutzelementen (3, 4) je eines an zwei gegenüberliegenden Seiten des Handflächenschutzelementes (5) angeordnet ist, und/oder dass die Fingerschutzelemente (3, 4) an einem Ende offen und an ihrem dem offenen Ende gegenüberliegenden Ende geschlossen sind, wobei das offene Ende dem Handflächenschutzelement (5) zugewandt und das geschlossene Ende von dem Handflächenschutzelement (5) abgewandt ist.

6. Schutzeinrichtung (1) nach einem der vorhergehenden Ansprüche, **dadurch gekennzeichnet, dass** die Fingerschutzelemente (3, 4) jeweils eine Fingerschutzkappe (11) mit einer geschlossenen Oberseite (11a) und einer offenen Unterseite (11b) umfassen, bevorzugt, wobei die Fingerschutzkappen (11) zumindest im Wesentlichen die Form eines Hohlkegelstumpfes oder zumindest im Wesentlichen die Form eines einseitig geschlossenen Hohlzylinders aufweisen, und/oder wobei sich die Fingerschutzkappen (11) im Bereich ihrer geschlossenen Oberseiten (11a) durch eine größere Materialstärke auszeichnen als im verbleibenden Bereich.

7. Schutzeinrichtung (1) nach einem der vorhergehenden Ansprüche, **dadurch gekennzeichnet, dass** die Fingerschutzelemente (3, 4) jeweils einen Fingerschutzsteg (12) aufweisen, insbesondere, wobei der Fingerschutzsteg (12) des für den Daumen (6) bestimmten Fingerschutzelementes (3) eine geringere Länge aufweist als der Fingerschutzsteg (12) des für den weiteren Finger (7) bestimmten Fingerschutzelementes (4) und/oder wobei die Materialstärke der Fingerschutzstege (12) zumindest im Bereich der den Fingerkuppen zugeordneten vorderen Enden mindestens 1,0 mm, insbesondere mindestens 2 mm beträgt und/oder maximal 5,0 mm, insbesondere maximal 4,0 mm beträgt und bevorzugt 3,0 ± 0,5 mm beträgt.

8. Schutzeinrichtung (1) nach Anspruch 6 und 7, **dadurch gekennzeichnet,** sich der Fingerschutzsteg (12) jeweils an die offene Unterseite (11b) der Fingerschutzkappe (11) anschließt.

9. Schutzeinrichtung (1) nach einem der Ansprüche 6 oder 8, **dadurch gekennzeichnet, dass** an der Innenseite und/oder der Außenseite der Fingerschutzkappen (11) Noppen (13) vorgesehen sind.

10. Schutzeinrichtung (1) nach einem der Ansprüche 7 bis 9, **dadurch gekennzeichnet,**
**dass** die Materialstärke der Fingerschutzkappen (11) zumindest abschnittsweise mindestens 1,0 mm, insbesondere mindestens 2 mm beträgt und/oder dass die Materialstärke der Fingerschutzkappen (11) maximal 5,0 mm, insbesondere maximal 4,0 mm beträgt und bevorzugt 3,0 ± 0,5 mm beträgt, und/oder
**dass** der Innenraum der Fingerschutzkappen (11) insbesondere an der breitesten Stelle einen Durchmesser von 1,5 bis 3,0 cm aufweist, und/oder dass der Innenraum der Fingerschutzkappen (11) eine Länge im Bereich von 1,5 bis 6,0 cm aufweist, und/oder
**dass** die Fingerschutzkappen (11) eine Länge von wenigstens 2 cm, insbesondere wenigstens 2,5 cm und/oder von höchstens 4,0 cm, insbesondere höchstens 3,0 cm aufweisen.

11. Schutzeinrichtung (1) nach einem der vorhergehenden Ansprüche, **dadurch gekennzeichnet, dass** die Fingerschutzelemente (3, 4) ausschließlich über das Handflächenschutzelement (5) miteinander verbunden sind.

12. Schutzeinrichtung (1) nach einem der vorhergehenden Ansprüche, **dadurch gekennzeichnet,**
**dass** das Handflächenschutzelement (5) als flexible Matte ausgestaltet ist, und/oder
**dass** das Handflächenschutzelement (5) derart ausgestaltet und angeordnet ist, dass es sich im bestimmungsgemäß getragenen Zustand der Schutzeinrichtung (1), wenn die beiden Fingerschutzelemente (3, 4) jeweils auf einen Finger, insbesondere den Daumen (6) und Zeigefinger (7), eines Benutzers aufgesetzt sind, schützend vor der Handinnenfläche des Benutzers erstreckt.

13. Schutzeinrichtung (1) nach einem der vorhergehenden Ansprüche, **dadurch gekennzeichnet,**
**dass** das Handflächenschutzelement (5) eine Dicke von wenigstens 1,0 mm, insbesondere wenigstens 2 mm und/oder maximal 5,0 mm, insbesondere maximal 4 mm aufweist, wobei die Dicke bevorzugt 3,0 ± 0,5 mm beträgt, und/oder
**dass** das Handflächenschutzelement (5) eine Länge von mindestens 6,0 cm, insbesondere mindestens 7,0 cm und/oder von höchstens 11,0 cm, insbesondere höchstens 9,0 ± 0,5 cm aufweist.

14. Schutzeinrichtung (1) nach einem der vorhergehenden Ansprüche, **dadurch gekennzeichnet, dass** die Fingerschutzelemente (3, 4) und/oder das Handflächenschutzelement (5) wenigstens ein elastisch verformbares Material, insbesondere wenigsten ein Elastomer und/oder Silikon, umfassen oder daraus bestehen, wobei insbesondere das Material verstärkt ist und bevorzugt Verstärkungselemente in der Form von Streifen aus Metallblech, Kunststoff oder Verstärkungsfasern in das Material eingebettet sind.

15. Schutzeinrichtung (1) nach einem der vorhergehenden Ansprüche, **dadurch gekennzeichnet, dass** die Schutzeinrichtung (1) einteilig ausgestaltet ist, und/oder dass die Schutzeinrichtung (1) derart ausgestaltet ist, dass sie drei Finger, bevorzugt den Ringfinger (8), Mittelfinger (9) und kleinen Finger (10), und/oder den Handrücken (2a) und/oder das Handgelenk (2b) frei lässt.

## Claims

1. Protective device (1) for a hand (2), which comprises precisely two finger protection elements (3, 4) and a palm protection element (5) connecting the two finger protection elements (3, 4), wherein one finger protection element (3) is intended for the thumb (6) and the other finger protection element (4) is intended for a further finger, in particular the index finger (7), of a user, **characterized in that** the palm protection element (2) is designed to be planar, so that it only comes into contact with the palm of a user's hand, whereas the back of the user's hand remains free or essentially free, wherein the finger protection elements (3, 4) both lie closer to one end region (5b) of the palm protection element (5) than to another end region (5a) of the palm protection element (5) opposite the one end region (5b).

2. Protective device (1) according to claim 1, **characterized in that** the finger protection elements (3, 4) both lie directly at the one end region (5b) of the palm protection element (5), and/or wherein the other end region (5a) is a free end region of the palm protection element (5).

3. Protective device (1) according to claim 2, **characterized in that** the palm protective element (5) has a preferably free end region with a U-shaped outer contour, tip region (5a), and an end region opposite the tip region (5a), root region (5b), the finger protective elements (3, 4) both lying closer to the root region (5b) than to the tip region (5a), in particular, the finger protection elements (3, 4) both lying directly on the root region (5b), preferably, the root region (5b) terminating with an edge (5c), in particular a straight edge, opposite the tip region (5a), and the finger protection elements (3, 4) extending up to this edge (5c).

4. Protective device (1) according to claim 2 or 3, **characterized in that** the finger protection elements (3, 4) are arranged laterally of the palm protection element (5) with respect to the two opposite end regions (5a, 5b), preferably projecting laterally from the palm protection element (5).

5. Protective device (1) according to claim 4, **characterized in that** one of the finger protection elements (3, 4) is arranged on each of two opposite sides of the palm protection element (5), and/or **in that** the finger protection elements (3, 4) are open at one end and closed at their end opposite the open end, the open end facing the palm protection element (5) and the closed end facing away from the palm protection element (5).

6. Protective device (1) according to one of the preceding claims, **characterized in that** the finger protection elements (3, 4) each comprise a finger protection cap (11) with a closed upper side (11a) and an open lower side (11b), preferably wherein the finger protection caps (11) have at least substantially the shape of a hollow truncated cone or at least substantially the shape of a hollow cylinder closed on one side, and/or wherein the finger protection caps (11) are **characterized by** a greater material thickness in the region of their closed upper sides (11a) than in the remaining region.

7. Protective device (1) according to one of the preceding claims, **characterized in that** the finger protection elements (3, 4) each have a finger protection web (12), in particular, wherein the finger protection web (12) of the finger protection element (3) intended for the thumb (6) has a shorter length than the finger protection web (12) of the finger protection element (4) intended for the further finger (7) and/or wherein the material thickness of the finger protection webs (12), at least in the region of the front ends associated with the fingertips, is at least 1.0 mm, in particular at least 2 mm, and/or is at most 5.0 mm, in particular at most 4.0 mm, and is preferably 3.0 ± 0.5 mm.

8. Protective device (1) according to claim 6 and 7, **characterized in that** the finger protection web (12) is connected to the open underside (11b) of the finger protection cap (11).

9. Protective device (1) according to one of claims 6 or 8, **characterized in that** nubs (13) are provided on the inside and/or the outside of the finger protection caps (11).

10. Protective device (1) according to one of claims 7 to 9, **characterized in,**
**that** the material thickness of the finger protection caps (11) is at least 1.0 mm, in particular at least 2 mm, at least in sections, and/or in that the material thickness of the finger protection caps (11) is at most 5.0 mm, in particular at most 4.0 mm, and is preferably 3.0 ± 0.5 mm, and/or
**that** the interior of the finger protection caps (11) has a diameter of 1.5 to 3.0 cm, in particular at the widest point, and/or in that the interior of the finger protection caps (11) has a length in the range from 1.5 to 6.0 cm, and/or
in that the finger protection caps (11) have a length of at least 2 cm, in particular at least 2.5 cm, and/or of at most 4.0 cm, in particular at most 3.0 cm.

11. Protective device (1) according to one of the preceding claims, **characterized in that** the finger protection elements (3, 4) are connected to each other exclusively via the palm protection element (5).

12. Protective device (1) according to one of the preceding claims, **characterized in**
**that** the palm protection element (5) is designed as a flexible mat, and/or
**that** the palm protection element (5) is designed and arranged in such a way that, in the state in which the protective device (1) is worn as intended, when the two finger protection elements (3, 4) are each placed on a finger, in particular the thumb (6) and index finger (7), of a user, it extends protectively in front of the palm of the user's hand.

13. Protective device (1) according to one of the preceding claims, **characterized in**
**that** the palm protection element (5) has a thickness of at least 1.0 mm, in particular at least 2 mm, and/or a maximum of 5.0 mm, in particular a maximum of 4 mm, the thickness preferably being 3.0 ± 0.5 mm, and/or
**that** the palm protection element (5) has a length of at least 6.0 cm, in particular at least 7.0 cm and/or of at most 11.0 cm, in particular at most 9.0 ± 0.5 cm.

14. Protective device (1) according to one of the preceding claims, **characterized in that** the finger protection elements (3, 4) and/or the palm protection element (5) comprise or consist of at least one elastically deformable material, in particular at least one elastomer and/or silicone, wherein in particular the material is reinforced and preferably reinforcing elements in the form of strips of metal sheet, plastic or reinforcing fibers are embedded in the material.

15. Protective device (1) according to one of the preceding claims, **characterized in that** the protective device (1) is designed in one piece, and/or **in that** the protective device (1) is designed in such a way that it leaves three fingers, preferably the ring finger (8), middle finger (9) and little finger (10), and/or the back of the hand (2a) and/or the wrist (2b) free.

## Revendications

1. Dispositif de protection (1) pour une main (2), qui comprend précisément deux éléments de protection des doigts (3, 4) et un élément de protection de la paume (5) reliant les deux éléments de protection des doigts (3, 4), ou un élément de protection des doigts (3) est destiné au pouce (6) et l'autre élément de protection des doigts (4) est destiné à un autre doigt, en particulier l'index (7), d'un utilisateur, en particulier l'index (7) de l'utilisateur, et **caractérisé en ce que** l'élément de protection de la paume (5) est conçu pour être plan, de sorte qu'il n'entre en contact qu'avec la paume de la main de l'utilisateur, tandis que le dos de la main de l'utilisateur reste libre ou essentiellement libre, ou les éléments de protection des doigts (3, 4) sont tous deux plus proches d'une région d'extrémité (5b) de l'élément de protection de la paume (5) que d'une autre région d'extrémité (5a) de l'élément de protection de la paume (5) opposée à la région d'extrémité (5b).

2. Dispositif de protection (1) selon la revendication 1, **caractérisé en ce que** les éléments de protection des doigts (3, 4) sont tous deux situés directement au niveau de la région d'extrémité (5b) de l'élément de protection de la paume (5), et/ou dans lequel l'autre région d'extrémité (5a) est une région d'extrémité libre de l'élément de protection de la paume (5).

3. Dispositif de protection (1) selon la revendication 2, **caractérisé en ce que** l'élément de protection de la paume (5) a une région d'extrémité de préférence libre avec un contour extérieur en forme de U, région de pointe (5a), et une région d'extrémité opposée à la région de pointe (5a), région de racine (5b), les éléments de protection des doigts (3, 4) se trouvant tous deux plus près de la région de racine (5b) que de la région de pointe (5a), en particulier, les éléments de protection des doigts (3, 4) reposent tous deux directement sur la région de la racine (5b), de préférence, la région de la racine (5b) se terminant par un bord (5c), en particulier un bord droit, opposé à la région de la pointe (5a), et les éléments de protection des doigts (3, 4) s'étendant jusqu'à ce bord (5c).

4. Dispositif de protection (1) selon la revendication 2 ou 3, **caractérisé en ce que** les éléments de protection des doigts (3, 4) sont disposés latéralement par rapport à l'élément de protection de la paume (5) par rapport aux deux régions d'extrémité opposées (5a, 5b), de préférence en saillie latérale par rapport à l'élément de protection de la paume (5).

5. Dispositif de protection (1) selon la revendication 4, **caractérisé en ce qu'**un des éléments de protection des doigts (3, 4) est disposé sur chacun des deux côtés opposés de l'élément de protection de la paume (5), et/ou **en ce que** les éléments de protection des doigts (3, 4) sont ouverts à une extrémité et fermés à leur extrémité opposée à l'extrémité ouverte, l'extrémité ouverte faisant face à l'élément de protection de la paume (5) et l'extrémité fermée faisant face à l'opposé de l'élément de protection de la paume (5).

6. Dispositif de protection (1) selon l'une des revendications précédentes, **caractérisé en ce que** les éléments de protection des doigts (3, 4) comprennent chacun un capuchon de protection des doigts (11) avec un côté supérieur fermé (11a) et un côté inférieur ouvert (11b), de préférence dans lequel les capuchons de protection des doigts (11) ont au moins sensiblement la forme d'un cône tronqué creux ou au moins sensiblement la forme d'un cylindre creux fermé d'un côté, et/ou dans lequel les capuchons de protection des doigts (11) sont **caractérisés par** une épaisseur de matériau plus importante dans la région de leurs côtés supérieurs fermés (11a) que dans le reste de la région.

7. Dispositif de protection (1) selon l'une des revendications précédentes, **caractérisé en ce que** les éléments de protection des doigts (3, 4) comportent chacun une bande de protection des doigts (12), en particulier, dans lequel la bande de protection des doigts (12) de l'élément de protection des doigts (3) destiné au pouce (6) a une longueur plus courte que la bande de protection des doigts (12) de l'élément de protection des doigts (4) destiné à l'autre doigt (7) et/ou dans lequel l'épaisseur du matériau des bandes de protection des doigts (12), au moins dans la région des extrémités avant associées aux bouts des doigts, est d'au moins 1.0 mm, en particulier au moins 2 mm, et/ou est au maximum de 5,0 mm, en particulier au maximum de 4,0 mm, et est de préférence de 3,0 ± 0,5 mm.

8. Dispositif de protection (1) selon les revendications 6 et 7, **caractérisé en ce que** la bande de protection des doigts (12) est reliée à la face inférieure ouverte (11b) du capuchon de protection des doigts (11).

9. Dispositif de protection (1) selon l'une des revendications 6 ou 8, **caractérisé par la** présence de picots (13) à l'intérieur et/ou à l'extérieur des capuchons de protection des doigts (11).

10. Dispositif de protection (1) selon l'une des revendications 7 à 9, **caractérisé en ce que,**
l'épaisseur du matériau des capuchons de protection des doigts (11) est d'au moins 1,0 mm, en particulier d'au moins 2 mm, au moins en sections, et/ou l'épaisseur du matériau des capuchons de protection des doigts (11) est d'au plus 5,0 mm, en particulier d'au plus 4,0 mm, et est de préférence de 3,0 ± 0,5 mm, et/ou
l'intérieur des capuchons de protection des doigts (11) a un diamètre de 1,5 à 3,0 cm, en particulier au point le plus large, et/ou l'intérieur des capuchons de protection des doigts (11) a une longueur comprise entre 1,5 et 6,0 cm, et/ou en ce sens que les capuchons de protection des doigts (11) ont une longueur d'au moins 2 cm, en particulier d'au moins 2,5 cm, et/ou d'au plus 4,0 cm, en particulier d'au plus 3,0 cm.

11. Dispositif de protection (1) selon l'une des revendications précédentes, **caractérisé en ce que** les éléments de protection des doigts (3, 4) sont reliés entre eux exclusivement par l'élément de protection de la paume (5).

12. Dispositif de protection (1) selon l'une des revendications précédentes, **caractérisé en ce**
**que** l'élément de protection de la paume (5) est conçu comme un tapis flexible, et/ou
**que** l'élément de protection de la paume (5) est conçu et disposé de telle sorte que, dans l'état où le dispositif de protection (1) est porté comme prévu, lorsque les deux éléments de protection des doigts (3, 4) sont placés chacun sur un doigt, en particulier le pouce (6) et l'index (7), d'un utilisateur, il s'étend de manière protectrice devant la paume de la main de l'utilisateur.

13. Dispositif de protection (1) selon l'une des revendications précédentes, **caractérisé en ce**
**que** l'élément de protection de la paume (5) a une épaisseur d'au moins 1,0 mm, en particulier d'au moins 2 mm, et/ou d'un maximum de 5,0 mm, en particulier d'un maximum de 4 mm, l'épaisseur étant de préférence de 3,0 ± 0,5 mm, et/ou
l'élément de protection de la paume (5) a une longueur d'au moins 6,0 cm, en particulier d'au moins 7,0 cm et/ou d'au plus 11,0 cm, en particulier d'au plus 9,0 ± 0,5 cm.

14. Dispositif de protection (1) selon l'une des revendications précédentes, **caractérisé en ce que** les éléments de protection des doigts (3, 4) et/ou l'élément de protection de la paume (5) comprennent ou sont constitués d'au moins un matériau élastiquement déformable, en particulier au moins un élastomère et/ou du silicone, dans lequel en particulier le matériau est renforcé et, de préférence, des éléments de renforcement sous forme de bandes de tôle, de plastique ou de fibres de renforcement sont incorporés dans le matériau.

15. Dispositif de protection (1) selon l'une des revendications précédentes, **caractérisé en ce que** le dispositif de protection (1) est conçu en une seule pièce, et/ou **en ce que** le dispositif de protection (1) est conçu de manière à laisser libres trois doigts, de préférence l'annulaire (8), le majeur (9) et l'auriculaire (10), et/ou le dos de la main (2a) et/ou le poignet (2b).
